(19) Europäisches Patentamt
European Patent Office
Office européen des brevets

(11) Veröffentlichungsnummer: **0 044 413**
**B1**

# (12) EUROPÄISCHE PATENTSCHRIFT

(45) Veröffentlichungstag der Patentschrift: **15.02.84**

(51) Int. Cl.³: **C 07 C 33/04,** C 07 C 33/02, C 07 C 69/145, C 07 C 31/02, C 07 C 29/38, C 07 C 29/17, A 61 K 7/46

(21) Anmeldenummer: **81104694.5**

(22) Anmeldetag: **19.06.81**

## (54) 3,6-Dimethyl-oct-1-in-3-ole und -oct-1-en-3-ole sowie Derivate davon und ihre Verwendung als Riechstoffe und Verfahren zur Herstellung von 3,6-Dimethyl-octan-3-ol.

(30) Priorität: **18.07.80 DE 3027269**

(43) Veröffentlichungstag der Anmeldung: **27.01.82 Patentblatt 82/4**

(45) Bekanntmachung des Hinweises auf die Patenterteilung: **15.02.84 Patentblatt 84/7**

(84) Benannte Vertragsstaaten: **CH DE FR GB LI NL**

(56) Entgegenhaltungen:
**AT - B - 340 884**
**DE - A - 1 592 997**
**DE - B - 1 293 153**
**DE - B - 2 329 145**
**GB - A - 936 321**
**US - A - 2 908 722**

(73) Patentinhaber: **BASF Aktiengesellschaft, Carl-Bosch-Strasse 38, D-6700 Ludwigshafen (DE)**

(72) Erfinder: **Gramlich, Walter, Dr., Auf der Hoehe 11, D-6803 Edingen (DE)**
Erfinder: **Hoffmann, Werner, Dr., Ringstrasse 11 C, D-6701 Neuhofen (DE)**
Erfinder: **Hupfer, Leopold, Dr., Waltershoehe 3, D-6701 Friedelsheim (DE)**
Erfinder: **Meissner, Bernd, Dr., Dammweg 15, D-6900 Heidelberg (DE)**
Erfinder: **Paetsch, Juergen, Dr., Am Altenbach 30, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 044 413 B1

# 3,6-Dimethyl-oct-1-in-3-ole und -oct-1-en-3-ole sowie Derivate davon und ihre Verwendung als Riechstoffe und Verfahren zur Herstellung von 3,6-Dimethyl-octan-3-ol

Die Erfindung betrifft gegebenenfalls in 4,5-Stellung olefinisch ungesättigte 3,6-Dimethyl-oct-1-in-3-ole bzw. -oct-1-en-3-ole sowie deren Ester mit niedrigen Alkansäuren, deren Verwendung als Riechstoffe sowie ein Verfahren zur Herstellung von 3,6-Dimethyl-octan-3-ol über die neuen Alkohole. Die neuen Verbindungen haben die allgemeine Formel I

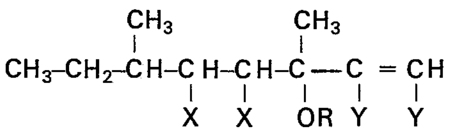 (I),

in der X und Y für H stehen oder aber jeweils beide X und/oder beide Y zusammen für eine weitere Bindung zwischen den sie tragenden C-Atomen stehen und R für H, $-CO-CH_3$; $-CO-C_2H_5$ oder $-CO-C_3H_7$ steht.

Aus der DE-A 1 592 997 sind die nach frischen Maiglöckchen riechenden Duftstoffe 3,7-Dimethyl-oct-6-en-1-in-3-ol und 3,7-Dimethyl-octa-1,6-dien-3-ol bekannt. Ester des 3,7-Dimethyl-octa-1,7-dien-3-ols mit aliphatischen oder aromatischen Carbonsäuren mit bis zu 8 C-Atomen sind Gegenstand der DE-B 1 293 153. Diese Ester weisen einen fruchtigen Geruch auf oder haben in ihren höheren Gliedern fixierende Eigenschaften in Duftstoffkompositionen. Weiterhin ist aus der GB-A 936 321 das 3,6-Dimethyl-octan-3-ol bekannt, das wegen seines frischen lavendelartigen Geruchs zu einem sehr wichtigen Duftstoff für Seifen und Waschmittel geworden ist.

Die Herstellung dieser Verbindung gemäss der GB-A 936 321 ist jedoch wirtschaftlich unbefriedigend, da man hierzu vom 3,6-Dimethyl-oct-4-in-3,6-diol ausgehen muss, dessen Hydrierung nur unter Einhaltung ganz bestimmter Reaktionsbedingungen zu überwiegenden Anteilen des gewünschten Octanols führt, meist jedoch ein Reaktionsgemisch verschiedener Hydrierungsprodukte liefert, in welchen das Octanol nur in mässiger Ausbeute vorliegt und aus dem es auch mit erheblichem Aufwand isoliert werden kann.

Der Erfindung lag daher die Aufgabe zugrunde, die Palette der Duftstoffe um neue Verbindungen mit neuartigen Geruchsnoten zu bereichern. Weiterhin war es Aufgabe der Erfindung, den besonders wichtigen bekannten Duftstoff 3,6-Dimethyl-octan-3-ol besser zugänglich zu machen.

Die eingangs definierten neuen Verbindungen der Formel I weisen interessante, vorwiegend blumige, holzige, krautige und fruchtige Geruchsnoten auf, die sich in charakteristischer Weise von den vorbekannten Verbindungen ähnlicher Struktur unterscheiden.

Besondere Bedeutung erlangen die neuen Verbindungen auch dadurch, dass über sie als Zwischenprodukte ein neuer und besonders vorteilhafter Weg zu dem in grossem Massstab als Duftstoff für Seifen und Waschmittel begehrten 3,6-Dimethyl-octan-3-ol eröffnet wird. Dies ist dadurch möglich, dass sie einerseits auf relativ einfache Weise aus gut zugänglichen Ausgangsverbindungen hergestellt werden können und andererseits auf einfache Weise in das gewünschte 3,6-Dimethyl-octan-3-ol überführt werden können.

Als Ausgangsstoffe für die Herstellung der erfindungsgemässen Verbindungen dienen 5-Methyl-hept-3-en-2-on und 5-Methyl-heptan-2-on, die auf einfache Weise durch Kondensation von 2-Methyl-butanal und Aceton und gegebenenfalls gleichzeitige oder nachträgliche Hydrierung der olefinischen Doppelbindung herstellbar sind. 2-Methyl-butanal seinerseits ist durch Hydroformylierung von 1- oder 2-Buten erhältlich.

Die Verbindungen der Formel I, in der R für H und die Y für eine weitere Bindung stehen, erhält man beispielsweise dadurch, dass man 5-Methyl-hept-3-en-2-on bzw. 5-Methyl-heptan-2-on, d.h. also Ketone der allgemeinen Formel II

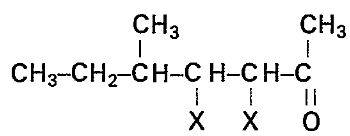 (II),

in der die X die oben angegebene Bedeutung haben, mit der Lösung eines Äthinylmagnesiumhalogenids in Tetrahydrofuran oder Dimethylether oder in Gegenwart von Katalysatoren mit Acetylen umsetzt.

Die Verbindungen der Formel I, in der R für H und die Y für H stehen, erhält man beispielsweise dadurch, dass man die Ketone der Formel II mit der Lösung eines Vinylmagnesiumhalogenids umsetzt oder aber indem man die Dreifachbindung der bei der Äthinylierung der Ketone der Formel II erhaltene Alkinole partiell hydriert. Diese Umsetzungen werden durch die folgenden Reaktionsgleichungen darstellt:

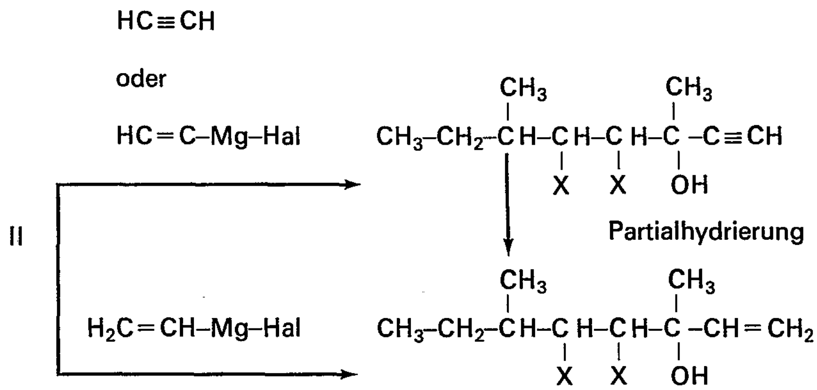


Die Veresterung der Hydroxylgruppe kann als Abschlussreaktion, aber auch vor der gegebenenfalls durchzuführenden Partialhydrierung vorgenommen werden.

Aus den Alkoholen der allgemeinen Formel I kann das als Riechstoff sehr begehrte 3,6-Dimethyl-3-hydroxy-octan auf einfache Weise durch katalytisches Hydrieren hergestellt werden.

Gegenstand der Erfindung ist dementsprechend auch ein Verfahren zur Herstellung von 3,6-Dimethyl-3-hydroxy-octan, das dadurch gekennzeichnet ist, dass man Ketone der allgemeinen Formel II

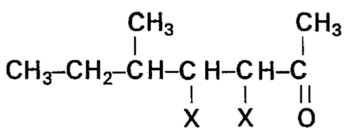 (II)

in der die X für H stehen oder aber beide X zusammen für eine weitere Bindung zwischen den sie tragenden C-Atomen stehen, mit der Lösung eines Ethinyl- oder Vinylmagnesiumhalogenids in einem etherischen Lösungsmittel, oder in Gegenwart von Katalysatoren mit Acetylen umsetzt und die dabei erhaltenen neuen Verbindungen der allgemeinen Formel Ia

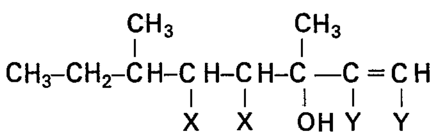 (Ia)

in der die X und die Y die oben angegebene Bedeutung haben in an sich bekannter Weise katalytisch hydriert.

Die Umsetzung der Ketone II zu den Alkoholen I bzw. Ia und dessen Hydrierung zum Octanol erfolgen alle in an sich bekannter Weise, so dass sich detaillierte Angaben über die Reaktionsbedingungen erübrigen.

Die Ethinylierung erfolgt beispielsweise entweder durch Umsetzung der Ketone der Formel II mit einer Lösung von Ethinylmagnesiumhalogeniden in Tetrahydrofuran oder Diethylether oder durch Umsetzung der Ketone II mit Acetylen in inerten organischen Lösungsmitteln in Gegenwart von Schwermetallacetyliden wie Kupferacetylid und Silberacetylid oder in Gegenwart von basisch reagierenden Katalysatoren wie Natrium- oder Kaliumacetylid, den Oxiden, Hydroxiden, Alkoholaten, Hydriden oder Amiden der Alkali- oder Erdalkalimetalle oder in Gegenwart von quartären Ammoniumgruppen enthaltenden Anionenaustauschern (s. z.B. die belgische Patentschrift 725 275).

Die Ethinylierung wird bei Temperaturen von −20 bis +70 °C, vorzugsweise 10 bis +50 °C und unter Drücken von Normaldruck bis etwa 30 atm durchgeführt. Die Aufarbeitung und Isolierung der Reaktionsprodukte erfolgt durch Hydrolyse und fraktionierte Destillation der organischen Phase.

Die partielle Hydrierung der erhaltenen Alkinole kann in Lösungsmitteln aber auch ohne Lösungsmittel erfolgen.

Als Katalysatoren sind Palladium-Träger-Katalysatoren, die 0,01 bis 5 Gewichtsprozent Palladium enthalten, besonders geeignet.

Als Träger seien insbesondere Calciumcarbonat, Bariumhydroxid, Aluminiumoxid und Siliciumdioxid genannt. Zur Erhöhung der Selektivität empfiehlt es sich, die genannten Katalysatoren z.B. gemäss der deutschen Patentschrift 1 115 238 durch Behandeln mit Zink- oder Bleiionen zu desaktivieren.

Die Hydrierung wird im allgemeinen bei Normaldruck oder einem Wasserstoffüberdruck von 0,1 bis 1 bar und bei Temperaturen von etwa 0 bis 80 °C, vorzugsweise 15 bis 35 °C durchgeführt. Die Reaktionsprodukte werden durch Filtration und Destillation isoliert.

Die Umsetzung der Ketone der Formel II mit der Lösung eines Vinylmagnesiumhalogenids erfolgt auf die für Grignard-Reaktionen übliche Weise. Mit Vorteil arbeitet man mit etwa 1 bis 2 molaren Lösungen des Vinylmagnesiumchlorids in etherischen Lösungsmitteln, wie Diethylether, Tetrahydrofuran oder Diethylenglykoldimethylether und bei Temperaturen von −20 bis +50 °C.

Auch die Einführung der Estergruppe kann auf verschiedene bekannte Methoden erfolgen.

So kann man die bei der Ethinylierung erhaltenen Alkinole in Gegenwart katalytischer Mengen von Mineralsäuren wie Schwefelsäure oder Phosphorsäure mit Carbonsäureanhydriden verestern und gewünschtenfalls die erhaltenen Ester partiell hydrieren.

Die bei der Vinylierung erhaltenen Alkenole kann man vorteilhaft in Gegenwart eines Säurefängers wie Trimethylamin oder Dimethylanilin mit einem Acylhalogenid der Formel R-Halogen

umsetzen oder aber in Gegenwart von Natriummethylat oder Kalium-tert.-butylat mit tert.-Butylacetat, -propionat oder -butyrat umestern.

Die katalytische Hydrierung der Alkohole der Formel Ia zu 3,6-Dimethyl-octan-3-ol erfolgt in üblicher Weise und mit üblichen Hydrierkatalysatoren. Genannt seien beispielsweise Palladium- oder Platinkatalysatoren.

Besonders geeignet sind Palladiumträgerkatalysatoren, wie $Pd/SiO_2$, $Pd/Al_2O_3$, Pd/Kohle und ähnliche. Zweckmässigerweise arbeitet man in einem unter den Reaktionsbedingungen inerten Lösungsmittel, wie Methanol, Essigester oder Tetrahydrofuran, bei Temperaturen von 20 bis 120 °C und Wasserstoffdrücken von 0 bis 50 bar.

Die Verfahrensprodukte sind alle durch Destillation in reiner Form gewinnbar.

Die erfindungsgemässen 3,6-Dimethyl-oct-1-in-3-ole und -oct-1-en-3-ole sowie deren Ester sind wertvolle Riechstoffe, die sich vorwiegend zur Herstellung von blumigen, krautigen und fruchtigen Kompositionen eignen. Darüberhinaus eröffnen sie einen neuen und besonders vorteilhaften Weg zur Synthese des als Duftstoff für Seifen und Waschmitteln sehr begehrten 3,6-Dimethyl-octan-3-ols.

Beispiel 1

3,6-Dimethyl-oct-1-in-3-ol (1c)

Zu 1 l einer 1,1 molaren Lösung von Ethinylmagnesiumchlorid in Tetrahydrofuran werden unter Rühren bei 10 °C innerhalb 1 Stunde 128 g (1 Mol) 5-Methyl-heptan-2-on gegeben. Man lässt etwa 1 Stunde nachreagieren und arbeitet dann das Reaktionsgemisch auf. Dazu gibt man unter Eiskühlung solange 2 n-Schwefelsäure zu, bis zwei klare Phasen vorhanden sind. Die organische Phase wird dann abgetrennt, gegebenenfalls mit Wasser neutral gewaschen und das Tetrahydrofuran bei 50 °C und 67 mbar abdestilliert. Der Rückstand wird fraktioniert destilliert. Man erhält 143 g Hauptlauf vom Siedepunkt Kp = 50–52 °C bei 0,01 mbar; $n_D^{25} = 1,4411$. Das entspricht einer Ausbeute von 93%. Geruch: vorwiegend blumig, holzig, krautig, minzig, im Nachgeruch würzig, schwach animalisch.

Beispiel 2

3,6-Dimethyl-oct-1-en-3-ol (1 d)

154 g (1 Mol) 3,6-Dimethyl-oct-1-in-3-ol werden in 100 ml Methanol gelöst, mit 1 g eines 0,1%igen Palladium/Kalziumcarbonat-Katalysators, der mit Zinkionen vergiftet wurde, versetzt und bei 20 °C und Normaldruck Wasserstoff eingeleitet. Die Hydrierung ist nach Aufnahme von etwa 24 l Wasserstoff innerhalb von 6 Stunden beendet.

Man filtriert vom Katalysator ab, entfernt das Methanol durch Abdestillieren bei 50 °C und 67 mbar und fraktioniert das Verfahrensprodukt. Dabei werden 146 g Hauptlauf vom Siedepunkt Kp = 46 bis 48 °C bei 0,1 mbar; $n_D^{25} = 1,4407$. Das entspricht einer Ausbeute von 94%. Geruch: vorwiegend blumig, holzig, krautig, im Nachgeruch minzig, süss.

Beispiel 3

3,6-Dimethyl-octan-3-ol

154 g (1 Mol) 3,6-Dimethyl-oct-1-in-3-ol werden mit 0,5 g eines 0,5%igen Palladium-Aluminiumoxid-Katalysators versetzt und bei 20 bis 50 °C und 0,5 bar Wasserstoffdruck hydriert. Nach Beendigung der Wasserstoffaufnahme (6 bis 8 Stunden) wird vom Katalysator abfiltriert und das Filtrat fraktioniert. Man erhält 145 g Hauptlauf vom Siedepunkt Kp = 42 bis 43 °C bei 0,01 mbar; $n_D^{25} = 1,4345$. Das entspricht einer Ausbeute von 91%. Geruch: frisch, blumig, lavendelartig.

Beispiel 4

3,6-Dimethyl-4-octen-1-in-3-ol

Zu 1 Liter einer 1,1 molaren Lösung von Ethinylmagnesiumchlorid in Tetrahydrofuran werden unter Rühren bei 10 °C innerhalb 1 Stunde 126 g (1 Mol) 5-Methyl-3-hepten-2-on gegeben. Man lässt etwa 1 Stunde nachreagieren und arbeitet dann das Reaktionsgemisch analog Beipsiel 1 auf. Man erhält 126 g 3,6-Dimethyl-4-octen-1-in-3-ol vom Siedepunkt Kp = 41 °C bei 0,05 mbar und $n_D^{25} = 1,4514$. Das entspricht einer Ausbeute von 83% d. Theorie. Geruch: holzig, krautig, fettig.

Beispiel 5

3,6-Dimethyl-octa-1,4-dien-3-ol

154 g (1 Mol) 3,6-Dimethyl-oct-1-in-4-en-3-ol werden in 100 ml Methanol gelöst, mit 1 g eines 0,1%igen Palladium-Kalziumcarbonat-Katalysators, der mit Zinkionen vergiftet wurde, versetzt und bei 15 bis 20 °C und Normaldruck Wasserstoff eingeleitet. Nach 6 Stunden arbeitet man analog Beispiel 2 auf. Man erhält 145 g 3,6-Dimethyl-octa-1,4-dien-3-ol vom Siedepunkt Kp = 38 bis 39 °C bei 0,01 mbar und $n_D^{25} = 1,4502$. Das entspricht einer Ausbeute von 93% d. Theorie. Geruch: holzig, fettig, minzig, schwach animalisch.

Beispiel 6

3,6-Dimethyl-3-acetoxy-oct-4-en-1-in

172 g (1,13 Mol) 3,6-Dimethyl-oct-4-en-1-in-3-ol werden mit 153 g Acetanhydrid, worin 0,5 g 85%ige Phosphorsäure gelöst sind, versetzt und bei 20 bis 25 °C ca. 16 Stunden gerührt. Danach wird das Reaktionsgemisch in ca. 500 ml-Ether aufgenommen, diese Lösung mit Wasser und Natriumcarbonatlösung neutral gewaschen und das Lösungsmittel bei 30 °C und 20 mm Hg abdestilliert. Das verbleibende Reaktionsprodukt wird fraktioniert, wobei 169 g des Acetats erhalten werden. Kp = 51 bis 52 °C bei 0,01 mbar; $n_D^{25} = 1,4606$. Das entspricht einer Ausbeute

von 77% der Theorie. Geruch: süsslich, Zimtalkohol-artig.

Beispiel 7

3,6-Dimethyl-3-acetoxy- octa-1,4-dien

87 g (0,5 Mol) 3,6-Dimethyl-3-ocetoxy-oct-4-en-1-in werden analog Beispiel 2 bei 15 bis 20 °C hydriert. Nach Aufarbeitung und Destillation erhält man 76 g 3,6-Dimethyl-3-acetoxy-octa-1,4-dien vom Siedepunkt Kp=105 bis 106 °C bei 15 mbar und $n_D^{25}$ =1,4550. Das entspricht einer Ausbeute von 87% der Theorie. Geruch: holzig, Zimtalkohol-artig.

Beispiel 8

3,6-Dimethyl-3-acetoxy-1-octin

154 g (1 Mol) 3,6-Dimethyl-1octin-3-ol werden analog Beispiel 6 mit Acetanhydrid verestert. Hierbei werden nach Aufarbeitung und Destillation 178 g des Acetats erhalten. Kp=44 bis 45 °C bei 0,01 mbar; $n_D^{25}$ =1,4352. Das entspricht einer Ausbeute von 91%. Geruch: blumig, cedernholzartig, schwach krautig.

**Patentansprüche**

1. 3,6-Dimethyl-oct-1-en-3-ole und 3,6-Dimethyl-oct-1-in-3-ole sowie deren Ester der allgemeinen Formel I

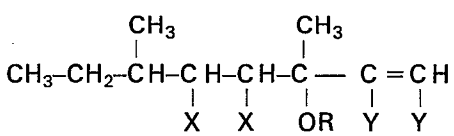

(I),

in der

X und Y für H stehen oder aber jeweils beide X und/oder beide Y zusammen für eine weitere Bindung zwischen den sie tragenden C-Atomen stehen und R für H; $-CO-CH_3$; $-CO-C_2H_5$ oder $-CO-C_3H_7$ steht.

2. 3,6-Dimethyl-oct-1-in-3-ol

3. 3,6-Dimethyl-oct-1-en-3-ol

4. Verwendung von 3,6-Dimethyl-oct-1-en-3-ol und dessen Derivaten gemäss Anspruch 1 als Riechstoffe.

5. Verfahren zur Herstellung von 3,6-Dimethyloctan-3-ol, dadurch gekennzeichnet, dass man Ketone der allgemeinen Formel II

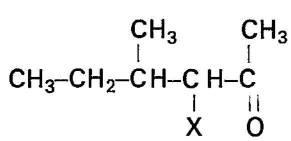

(II),

in der die X für H stehen oder aber beide X zusammen für eine weitere Bindung zwischen den sie tragenden C-Atomen stehen, in an sich bekannter Weise mit der Lösung eines Ethinyl- oder Vinylmagnesiumhalogenids in einem etherischen Lösungsmittel oder in Gegenwart von Katalysatoren mit Acetylen umsetzt und die dabei erhaltenen neuen Verbindungen der allgemeinen Formel Ia

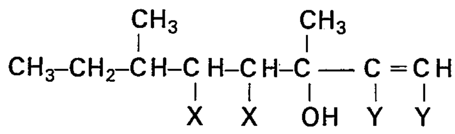

(Ia),

in der die X und die Y die oben angegebene Bedeutung haben in an sich bekannter Weise katalytisch hydriert.

**Revendications**

1. Diméthyl-3,6-octène-1 ols-3 et diméthyl-3,6-octyne-1 ols-3, ainsi que leurs esters, de formule générale I

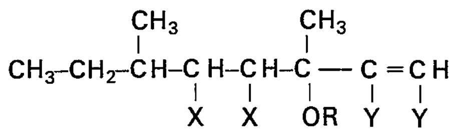

(I)

dans laquelle

X et Y sont mis pour H ou chacune des paires de symboles et/ou Y est mise pour une liaison supplémentaire entre les atomes de C qui les portent et R est mis pour H, $-CO-CH_3$, $-CO-C_2H_5$ ou $-CO-C_3H_7$.

2. Diméthyl-3,6 octyne-1 ol-3.

3. Diméthyl-3,6-octène-1 ol-3.

4. Utilisation de diméthyl-3,6 octène-1 ol-3 et de ses dérivés selon la revendication 1 comme matières odoriférantes.

5. Procédé pour la préparation du diméthyl-3,6 octanol-3, caractérisé en ce qu'on fait réagir, de façon connue en soi, des cétones de formule générale II

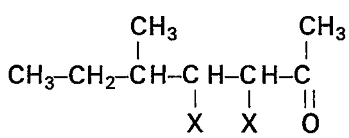

(II)

dans laquelle les symboles X sont mis pour H ou, ensemble, pour une liaison supplémentaire entre les atomes de C qui les portent, avec la solution d'un halogénure d'éthynyl-ou de vinyl-magnésium dans un solvant éthéré ou, en présence de catalyseurs, avec l'acétylène, et on hydrogène catalytiquement, de façon connue en soi, les nouveaux composés de formule générale Ia ainsi obtenus

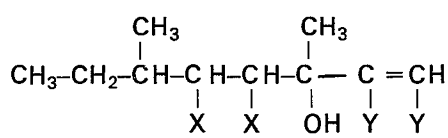

(Ia)

dans laquelle X et Y ont les significations données ci-dessus.

**Claims**

1. 3,6-Dimethyl-oct-1-en-3-ols and 3,6-dimethyl-oct-1-yn-3-ols and their esters, of the general formula I

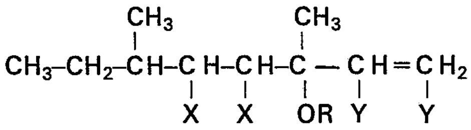

(I)

where X and Y are H or the two X's and/or the two Y's together are a further bond between the carbon atoms on which they are present, and R is H, $-CO-CH_3$, $-CO-C_2H_5$ or $-CO-C_3H_7$.

2. 3,6-Dimethyl-oct-1-yn-3-ol.

3. 3,6-Dimethyl-oct-1-en-3-ol.

4. Use of 3,6-dimethyl-oct-1-en-3-ol and its derivatives as claimed in claim 1 as scents.

5. A process for the preparation of 3,6-dimethyl-octan-3-ol, wherein a ketone of the general formula II

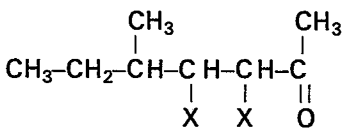

(II)

where the X's are H or the two X's together are a further bond between the carbon atoms on which they are present, is reacted in a conventional manner with a solution of an ethynyl-magnesium halide or vinyl-magnesium halide in an ether solvent, or with acetylene in the presence of a catalyst, and the resulting novel compound of the general formula Ia

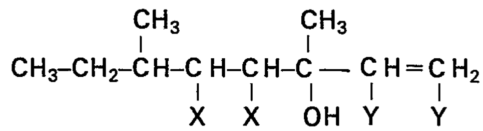

(Ia)

where the X's and the Y's have the above meanings, is catalytically hydrogenated in a conventional manner.